(19) **Europäisches Patentamt**
**European Patent Office**
**Office européen des brevets**

(11) **EP 4 233 677 A1**

(12) **EUROPEAN PATENT APPLICATION**
published in accordance with Art. 153(4) EPC

(43) Date of publication:
**30.08.2023 Bulletin 2023/35**

(21) Application number: **20958659.3**

(22) Date of filing: **21.10.2020**

(51) International Patent Classification (IPC):
***A61B 1/00*** (2006.01)

(52) Cooperative Patent Classification (CPC):
**A61B 1/00**

(86) International application number:
**PCT/JP2020/039529**

(87) International publication number:
**WO 2022/085106 (28.04.2022 Gazette 2022/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **NEC Corporation**
  **108-8001 Tokyo (JP)**
• **Olympus Medical Systems Corp.**
  **Tokyo 192-8507 (JP)**

(72) Inventors:
• **YOSHIOKA, Masaru**
  **Tokyo 108-8001 (JP)**

• **KAMIJO, Kenichi**
  **Tokyo 108-8001 (JP)**
• **MANO, Mieko**
  **Tokyo 108-8001 (JP)**
• **KOBAYASHI, Katsuhiko**
  **Tokyo 108-8001 (JP)**
• **IKEDA, Yuichi**
  **Hachioji-shi, Tokyo 192-8507 (JP)**
• **FUJITA, Hiromasa**
  **Hachioji-shi, Tokyo 192-8507 (JP)**

(74) Representative: **Betten & Resch**
**Patent- und Rechtsanwälte PartGmbB**
**Maximiliansplatz 14**
**80333 München (DE)**

(54) **ENDOSCOPE OPERATION ASSISTANCE DEVICE, CONTROL METHOD, COMPUTER-READABLE MEDIUM, AND PROGRAM**

(57) An endoscope operation support apparatus (2000) acquires a shape data sequence (50) representing a temporal change of shape data of an endoscope scope (40). The endoscope operation support apparatus (2000) determines an insertion method used for insertion of the endoscope scope (40) using the shape data sequence (50). The endoscope operation support apparatus (2000) outputs insertion method information (20) regarding the determined insertion method.

Fig. 1

EP 4 233 677 A1

## Description

### Technical Field

The present disclosure relates to a method for supporting an operation of an endoscope.

### Background Art

Devices that support surgery, examination, and the like using an endoscope have been developed. For example, Patent Literature 1 discloses a technology of estimating a current state regarding an endoscope operation and outputting auxiliary information according to the situation. As an example of a current state regarding the endoscope operation, for example, "inserting without a large problem", "keeping excessively close", and the like are disclosed. Furthermore, the auxiliary information indicates an operation of the endoscope effective for solving the problem of the current state.

### Citation List

### Patent Literature

Patent Literature 1: International Patent Publication No. WO2018/235185

### Summary of Invention

### Technical Problem

Information to support an endoscope operation is not limited to the above-described information. An object of the present disclosure is to provide a technology for presenting new information to support an endoscope operation.

### Solution to Problem

According to an aspect of the present disclosure, there is provided an endoscope operation support apparatus including: an acquisition unit configured to acquire a shape data sequence representing a temporal change of shape data of an endoscope scope; a determination unit configured to determine an insertion method used for insertion of the endoscope scope based on the shape data sequence; and an output unit configured to output insertion method information regarding the determined insertion method.

According to another aspect of the present disclosure, there is provided a control method executed by a computer. The control method includes: an acquisition step of acquiring a shape data sequence representing a temporal change of shape data of an endoscope scope; a determining step of determining an insertion method used for insertion of the endoscope scope based on the shape data sequence; and an output step of outputting insertion method information regarding the determined insertion method.

According to still another aspect, there is provided a computer readable medium storing a program, the program causing a computer to execute the control method of the present invention.

### Advantageous Effects of Invention

According to the present disclosure, there is provided a technology for presenting new information to assist an endoscope operation.

### Brief Description of Drawings

Fig. 1 is a diagram illustrating an overview of an operation of an endoscope operation support apparatus according to a first example embodiment.
Fig. 2 is a block diagram illustrating a functional configuration of the endoscope operation support apparatus according to the first example embodiment.
Fig. 3 is a block diagram illustrating a hardware configuration of a computer that implements the endoscope operation support apparatus.
Fig. 4 is a diagram illustrating a specific example of a use environment of an endoscope operation support apparatus.

For example, endoscope operation

Fig. 5 is a flowchart illustrating a flow of processing executed by the endoscope operation support apparatus according to the first example embodiment.

Fig. 6 is a diagram illustrating a configuration of a shape data sequence in a table format.

Fig. 7 is a diagram conceptually illustrating a method of computing likelihood using Equation (1).

Fig. 8 is a diagram illustrating insertion method information.

**Example Embodiment**

Hereinafter, example embodiments of the present disclosure will be described in detail with reference to the drawings. In the drawings, the same or corresponding elements are denoted by the same reference numerals, and repeated description is omitted as necessary for clarity of description. In addition, unless otherwise described, predetermined values such as predetermined values and thresholds are stored in advance in a storage device accessible from a device using the values.

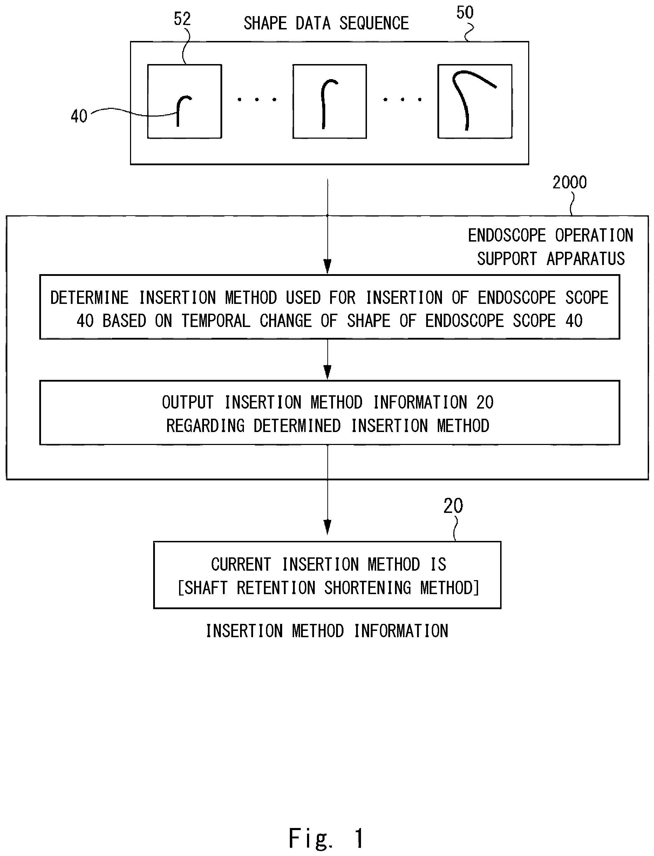

Fig. 1 is a diagram illustrating an overview of an operation of an endoscope operation support apparatus 2000 according to a first example embodiment. Here, Fig. 1 is a diagram for facilitating understanding of the overview of the endoscope operation support apparatus 2000, and the operation of the endoscope operation support apparatus 2000 is not limited to that illustrated in Fig. 1.

There are various types of operation methods (hereinafter, referred to as an insertion method or a procedure pattern) for inserting a scope of an endoscope into a body. Examples thereof include a shaft retention and shortening method, a push method, and a method of forming an $\alpha$ loop. The endoscope operation support apparatus 2000 determines an insertion method used for inserting an endoscope scope 40, and outputs insertion method information 20 which is information regarding the determined insertion method.

Here, the insertion of the endoscope scope 40 is performed while the shape of the endoscope scope 40 is variously changed. Then, how the shape of the endoscope scope 40 is changed differs depending on the insertion method. Therefore, it is possible to determine the insertion method used based on a temporal change in the shape of the endoscope scope 40.

Therefore, the endoscope operation support apparatus 2000 acquires a shape data sequence 50 representing the shape of the endoscope scope 40 in time series. Next, the endoscope operation support apparatus 2000 determines an insertion method used to insert the endoscope scope 40 using the shape data sequence 50. Then, the endoscope operation support apparatus 2000 generates and outputs insertion method information 20 regarding the determined insertion method. For example, the insertion method information 20 indicates the name of the determined insertion method.

<Example of Operation and Effect>

As described above, the endoscope operation support apparatus 2000 according to the present example embodiment determines the insertion method used for the insertion of the endoscope scope 40 using the shape data sequence 50 representing the temporal change in the shape of the endoscope scope 40, and outputs the insertion method information 20 regarding the insertion method. By using the insertion method information 20, an operator or the like of the endoscope scope 40 can grasp the insertion method used for inserting the endoscope scope 40.

For example, an actual motion of the endoscope scope 40 may be different from the motion intended by the operator. Therefore, even when the operator intends to perform a certain insertion method, the insertion method may not be actually performed. By using the endoscope operation support apparatus 2000 of the present example embodiment, the operator of the endoscope scope 40 can easily grasp whether or not the intended insertion method has been performed. In a case where the intended insertion method has been performed, the operator can continue the operation of the endoscope scope 40 with confidence after grasping that the intended insertion method has been performed. In addition, in a case where the intended insertion method has not been performed, the operator can grasp that the intended insertion method has not been performed and then modify the operation to perform the intended insertion method.

Furthermore, when the insertion method information 20 is stored in the storage device, it is possible to leave a record of the insertion method used for the operation of the endoscope scope 40. By keeping such a record, it is possible to use it for the operation of future endoscopic surgery, endoscopic examination, and the like. For example, by keeping a record of the insertion method used in the endoscopic surgery performed by an experienced doctor or the like, the technique of the experienced doctor can be shared with other doctors. In another example, by keeping the record of the used insertion method in association with a medical record, it can be used for explanation to the patient. Furthermore, the endoscope operation support apparatus 2000 may be configured to perform an operation guide for the operator based on information of the determined insertion method. This enables an operation guide corresponding to the insertion method that the operator intends to perform.

Hereinafter, the endoscope operation support apparatus 2000 according to the present example embodiment will be described in more detail.

<Example of Functional Configuration>

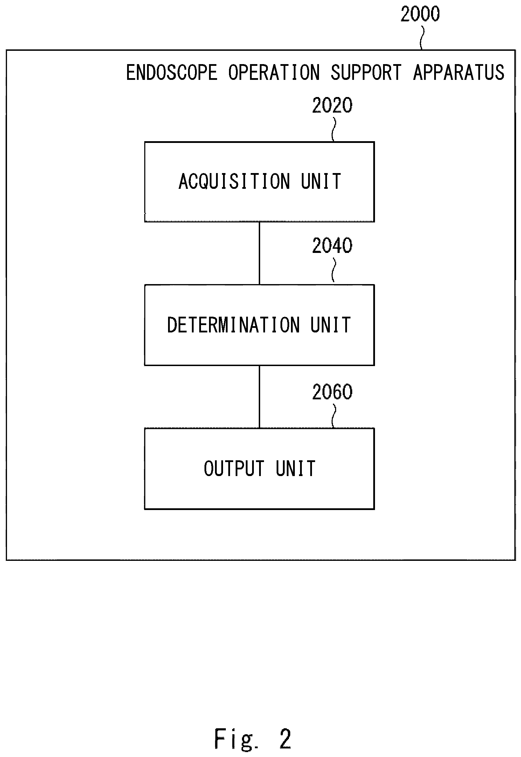

Fig. 2 is a block diagram illustrating a functional configuration of the endoscope operation support apparatus 2000 according to the first example embodiment. The endoscope operation support apparatus 2000 includes an acquisition unit 2020, a determination unit 2040, and an output unit 2060. The acquisition unit 2020 acquires the shape data sequence 50. The determination unit 2040 uses the shape data sequence 50 to determine the insertion method used for insertion of the endoscope scope 40. The output unit 2060 outputs insertion method information 20 regarding the determined insertion method.

<Example of Hardware Configuration>

Each functional component of the endoscope operation support apparatus 2000 may be realized by hardware (for example, a hard-wired electronic circuit or the like) that realizes each functional component, or may be realized by a combination of hardware and software (for example, a combination of an electronic circuit and a program for controlling the electronic circuit or the like). Hereinafter, a case where each functional component of the endoscope operation support apparatus 2000 is realized by a combination of hardware and software will be further described.

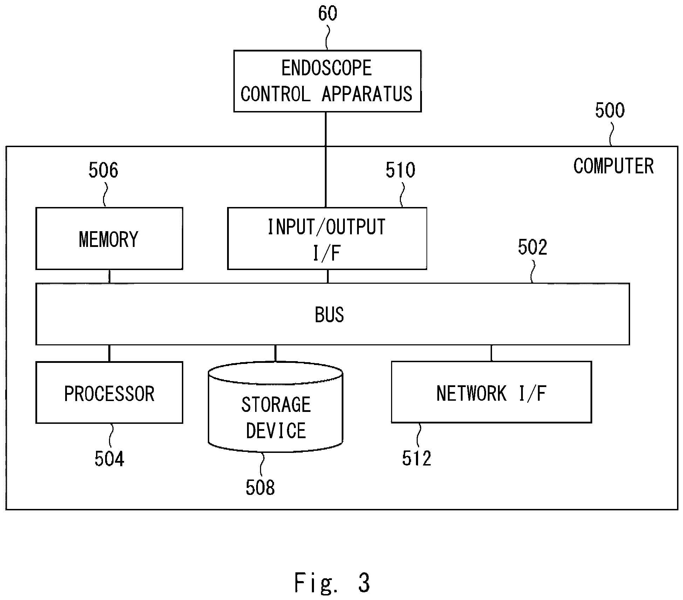

Fig. 3 is a block diagram illustrating a hardware configuration of a computer 500 that implements the endoscope operation support apparatus 2000. The computer 500 is any computer. For example, the computer 500 is a stationary computer such as a personal computer (PC) or a server machine. In another example, the computer 500 is a portable computer such as a smartphone or a tablet terminal. The computer 500 may be a special-purpose computer designed to realize the endoscope operation support apparatus 2000, or may be a general-purpose computer.

For example, by installing a predetermined application in the computer 500, each function of the endoscope operation support apparatus 2000 is realized in the computer 500. The application includes a program for realizing the functional component of the endoscope operation support apparatus 2000.

The computer 500 includes a bus 502, a processor 504, a memory 506, a storage device 508, an input/output interface 510, and a network interface 512. The bus 502 is a data transmission path for the processor 504, the memory 506, the storage device 508, the input/output interface 510, and the network interface 512 to transmit and receive data to and from each other. However, a method of connecting the processor 504 and the like to each other is not limited to the bus connection.

The processor 504 is various processors such as a central processing unit (CPU), a graphics processing unit (GPU), or a field-programmable gate array (FPGA). The memory 506 is a primary storage device realized by using a random access memory (RAM) or the like. The storage device 508 is a secondary storage device realized by using a hard disk, a solid state drive (SSD), a memory card, a read only memory (ROM), or the like.

The input/output interface 510 is an interface for connecting the computer 500 and an input/output device. For example, an endoscope control apparatus 60 to be described later is connected to the input/output interface 510. In another example, an input device such as a keyboard and an output device such as a display device are connected to the input/output interface 510.

The network interface 512 is an interface for connecting the computer 500 to a network. The network may be a local area network (LAN) or a wide area network (WAN).

The storage device 508 stores a program (program for realizing the above-described application) for realizing each functional component of the endoscope operation support apparatus 2000. The processor 504 implements each functional component of the endoscope operation support apparatus 2000 by reading and executing the program in the memory 506.

The endoscope operation support apparatus 2000 may be realized by one computer 500 or may be realized by a plurality of computers 500. In the latter case, the configurations of the computers 500 do not need to be the same, and can be different from each other.

<Example of Use Environment of Endoscope Operation Support Apparatus 2000>

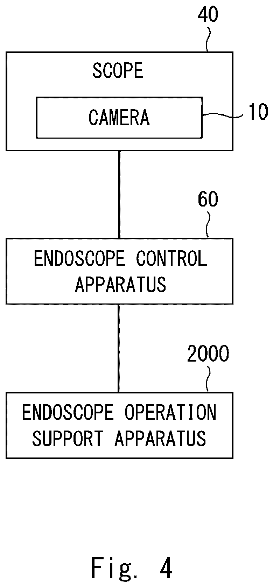

Fig. 4 is a diagram illustrating a specific example of a use environment of the endoscope operation support apparatus 2000. For example, the endoscope operation support apparatus 2000 is used together with the endoscope scope 40 and the endoscope control apparatus 60. The endoscope scope 40 is a scope to be inserted into the body, and is provided with a camera 10. By viewing video data generated by the camera 10, the state in the body can be visually recognized.

The endoscope control apparatus 60 is a control apparatus used when surgery or examination is performed using the endoscope scope 40. For example, the endoscope control apparatus 60 generates video data to be viewed, by performing various processing on video data obtained from a camera provided in the endoscope scope 40, and outputs the generated video data. Examples of processing on the video data include processing of adjusting color or brightness of the video data and processing of superimposing various types of information on the video data.

For example, the endoscope operation support apparatus 2000 acquires various types of information via the endoscope control apparatus 60. For example, the endoscope operation support apparatus 2000 acquires still image data or video data (hereinafter, these are collectively referred to as image data) generated by the camera 10 from the endoscope control apparatus 60.

Note that the data obtained from the endoscope scope 40 is not limited to the image data generated by the camera 10. For example, the endoscope scope 40 may be provided with elements for obtaining three-dimensional coordinates for each of a plurality of locations of the endoscope scope 40. In this case, the endoscope control apparatus 60 determines the three-dimensional coordinates of each of the plurality of locations of the endoscope scope 40 by using the element. By using the three-dimensional coordinates for the plurality of locations of the endoscope scope 40 obtained in this manner, it is possible to grasp the shape of the endoscope scope 40.

<Flow of Processing>

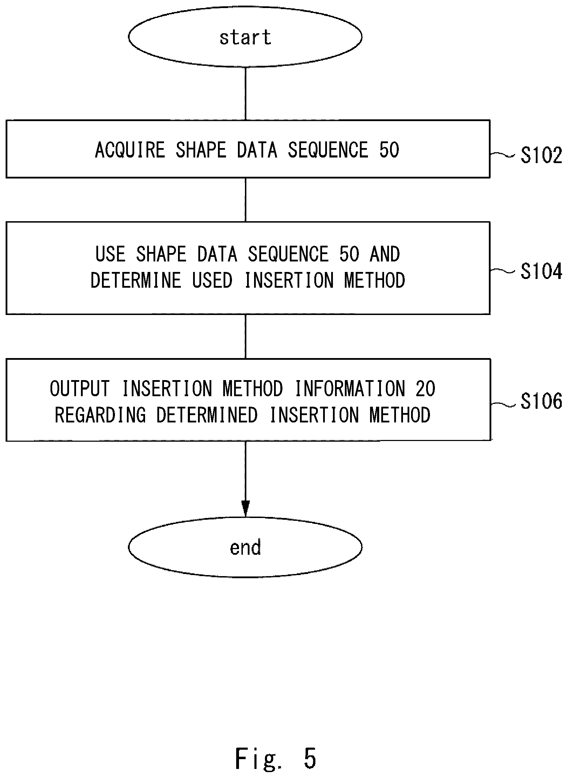

Fig. 5 is a flowchart illustrating a flow of processing executed by the endoscope operation support apparatus 2000 according to the first example embodiment. The acquisition unit 2020 acquires the shape data sequence 50 (S102). The determination unit 2040 determines the insertion method being used using the shape data sequence 50 (S104). The determination unit 2040 outputs the insertion method information 20 regarding the determined insertion method (S106).

<Shape Data Sequence 50>

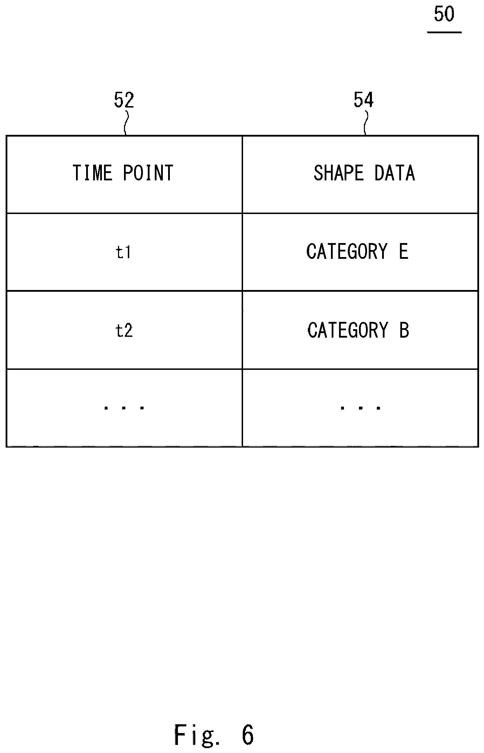

The shape data sequence 50 is data representing the shape of the endoscope scope 40 in time series. For example, for each of a plurality of times, the shape data sequence 50 indicates the shape of the endoscope scope 40 at that time. Fig. 6 is a diagram illustrating a configuration of the shape data sequence 50 in a table format. The shape data sequence 50 in Fig. 6 includes a time point 51 and shape data 52. Each record in the shape data sequence 50 indicates that the shape of the endoscope scope 40 was the shape indicated in the shape data 52 at the time point indicated by the time point 51. The time point 51 may be represented by time or may be represented by other than time. In the latter case, for example, the time point 51 indicates a relative numerical value assigned to each piece of shape data 52 in chronological order, such as a sequence number.

There are various ways of representing the shape of the endoscope scope 40. For example, the shape of the endoscope scope 40 is represented by a combination of three-dimensional coordinates of each of a plurality of specific locations of the endoscope scope 40. In another example, the shape of the endoscope scope 40 may be represented by one of a plurality of predetermined shape categories. For example, as the shape category, various categories such as "straight line ", "small curve", "large curve", or "abdomen protrusion" can be defined. In Fig. 6, the shape data 52 indicates a shape category.

Which shape category the shape of the endoscope scope 40 belongs to can be determined using, for example, the three-dimensional coordinates of each of the plurality of specific locations of the endoscope scope 40 described above. For example, an identification model (hereinafter, the shape identification model) is used to determine the shape category. The shape identification model can be realized by various types of models such as a neural network or a support vector machine.

The shape identification model is trained in advance so as to output a label representing a shape category of the endoscope scope 40 in response to input of three-dimensional coordinates of each of a plurality of specific locations of the endoscope scope 40. For each of a plurality of time points, by inputting the three-dimensional coordinates of each of a plurality of specific locations of the endoscope scope 40 at that time point to the shape identification model, it is possible to determine the shape category of the endoscope scope 40 at that time point. Note that the training of the shape identification model can be performed by using training data including a combination of "three-dimensional coordinates of each of a plurality of specific locations of the endoscope scope 40, and a label representing a ground-truth shape category".

<Acquisition of Shape Data Sequence 50: S102>

The acquisition unit 2020 acquires the shape data sequence 50 (S102). There are various methods for the acquisition unit 2020 to acquire the shape data sequence 50. For example, the acquisition unit 2020 acquires the shape data sequence 50 stored in a storage device accessible from the acquisition unit 2020 by accessing the storage device.

The storage device may be provided inside the endoscope operation support apparatus 2000 or may be provided outside the endoscope operation support apparatus 2000. In another example, the acquisition unit 2020 may acquire the shape data sequence 50 by receiving the shape data sequence 50 transmitted from another device.

In a case where the shape data sequence 50 represents the shape of the endoscope scope 40 by the shape category, it is necessary to perform processing of determining the shape category of the endoscope scope 40 at each time point. This processing may be performed by the endoscope operation support apparatus 2000 or may be performed by an apparatus other than the endoscope operation support apparatus 2000. In the former case, the acquisition unit 2020 acquires the shape data sequence 50 generated inside the endoscope operation support apparatus 2000. For example, in this case, the shape data sequence 50 is stored in a storage device inside the endoscope operation support apparatus 2000. Meanwhile, in a case where the shape data sequence 50 is generated by an apparatus other than the endoscope operation support apparatus 2000, for example, the acquisition unit 2020 acquires the shape data sequence 50 by accessing the device that has generated the shape data sequence 50 or receives the shape data sequence 50 transmitted from the device.

Here, the insertion method used may vary over time. Therefore, for example, it is preferable that the endoscope operation support apparatus 2000 acquires the shape data sequence 50 representing the temporal change of the shape data 52 in a period of a predetermined length (hereinafter, the unit period), and determines the insertion method represented by the shape data sequence 50. In this case, the endoscope operation support apparatus 2000 may acquire the shape data sequence 50 divided for each unit period in advance, or may acquire a plurality of pieces of shape data 52 not divided for each unit period.

In the latter case, for example, the acquisition unit 2020 obtains the shape data sequence 50 for each unit period by dividing the plurality of pieces of acquired shape data 52 for each unit period in chronological order. However, the unit periods adjacent to each other may partially overlap each other. For example, in a case where the length of the unit period is 10 and the length of the overlapping section is 4, the first shape data sequence 50 includes the first to tenth shape data 52, and the second shape data sequence 50 includes the seventh to sixteenth shape data 52.

<Identification of Insertion Method: S104>

The determination unit 2040 determines the insertion method being used using the shape data sequence 50 (S104). For example, the determination unit 2040 uses the shape data sequence 50 to compute, for each one of a plurality of possible insertion methods, the likelihood of the insertion method is used. Then, the determination unit 2040 determines the insertion method being used from among the plurality of insertion methods based on the likelihood computed for each insertion method. Note that a method of computing the likelihood will be described later.

Various methods may be employed to determine the insertion method being used based on the likelihood computed for each insertion method. For example, the determination unit 2040 determines the insertion method with the maximum likelihood as the insertion method being used. In another example, the determination unit 2040 determines the plurality of candidates of the insertion method being used based on the likelihood computed for each insertion method, and determines the insertion method being used from among the plurality of candidates. For example, the determination unit 2040 determines the top n (n > 1) insertion methods with high likelihood as the candidate of the insertion method being used. In another example, the determination unit 2040 determines an insertion method whose likelihood is equal to or more than a threshold as the candidate of the insertion method being used.

Various methods can be employed as a method of determining the insertion method being used from among a plurality of candidates. For example, a priority is assigned in advance to each of the plurality of insertion methods. In this case, for example, the determination unit 2040 determines an insertion method having the highest priority among the candidate insertion methods as the insertion method being used. In another example, the determination unit 2040 corrects the likelihood by multiplying the computed likelihood by a weight based on the priority for each candidate insertion method. Note that the weight based on the priority is determined in advance as a larger value as the priority is higher. The determination unit 2040 determines an insertion method having the maximum corrected likelihood as the insertion method being used.

In another example, in a case where the shape data 52 indicates the shape category, an important shape category or a transition (permutation of two or more shape categories) between important shape categories are determined in advance for each of a plurality of insertion methods. In this case, the determination unit 2040 determines an insertion method in which an important shape category defined for the insertion method or a transition between important shape categories is included in the shape data sequence 50 among the insertion methods determined as the candidates as the insertion method being used.

For example, it is assumed that insertion methods X and Y are determined as candidates based on the likelihood. In addition, it is assumed that an important shape category defined for the insertion method X is B, and an important shape category defined for the insertion method Y is C. Then, it is assumed that the time series of the shape data 52 indicated by the shape data sequence 50 is "A, C, D, C, A". In this case, the shape data sequence 50 does not include the shape category B, which is an important shape category in the insertion method X, but includes the shape category C, which is an important shape category in the insertion method Y. Therefore, the determination unit 2040 determines the insertion method Y as the insertion method being used.

It is assumed that there are a plurality of insertion methods in which the shape category and the transition between the shape categories that are important as described above are included in the shape data sequence 50. In this case, for example, the determination unit 2040 determines an insertion method with high computed likelihood among the plurality of insertion methods as the insertion method being used. In another example, the determination unit 2040 may determine, as the insertion method being used, an insertion method in which more important shape categories or transitions between shape categories are included in the shape data sequence 50.

The determination unit 2040 may use the information regarding the operator of the endoscope scope 40 to determine the insertion method being used from among the candidate insertion methods. For example, information in which identification information of the operator is associated with an insertion method that can be used by the operator is prepared in advance. The determination unit 2040 determines, as the insertion method being used, an insertion method defined as the insertion method that can be used by the operator of the endoscope scope 40 among the candidate insertion methods. Note that, in a case where there are a plurality of candidate insertion methods defined as the insertion methods that can be used by the operator of the endoscope scope 40, for example, the determination unit 2040 determines the insertion method being used based on the likelihood, the height of the priority described above, and the like.

In another example, information representing a technical level (for example, years of experience or the number of times of experience in endoscopic examination or the like, evaluation by an experienced doctor, or the like) of the operator may be used as the information regarding the operator of the endoscope scope 40. In this case, for each one of the plurality of the insertion methods, an association with a technical level of the operator is defined (for example, "used by persons who have five or more years of experience", and the like). The determination unit 2040 determines the insertion method being used from among the plurality of candidates based on the relationship between the technical level of the operator and the insertion method.

The determination unit 2040 may determine the insertion method being used from among candidate insertion methods by using image data obtained from the camera 10 provided in the endoscope scope 40. For example, the determination unit 2040 extracts features from the image data obtained from the camera 10, and determines an insertion method that matches those features as the insertion method being used among the candidate insertion methods. Features extracted from the image data include, for example, a phenomenon (referred to as a red ball) in which the entire image becomes red due to pressing of a tip of the endoscope scope 40 against the wall of the internal organs, characteristic movement of the screen or operation of the endoscope scope 40, and the like.

The determination unit 2040 may compute the likelihood that each insertion method is used in each of a plurality of methods (for example, a method using an identification model and a method using a state transition model, which will be described later), and determine the insertion method being used based on the computation result. For example, the determination unit 2040 computes a statistical value (for example, an average value or a maximum value) of the likelihood computed by each of the plurality of methods for each insertion method, and determines the insertion method having the maximum statistical value as the insertion method being used.

In another example, the determination unit 2040 may determine the insertion method used for each method of computing the likelihood. For example, it is assumed that the insertion method determined using the identification model is the insertion method X and the insertion method determined using the state transition model is the insertion method Y. In this case, the determination unit 2040 determines the insertion method X and the insertion method Y as the insertion methods being used. In this case, it is preferable that the insertion method information 20 indicates the method (method of computing the likelihood) used to determine the insertion method and the insertion method determined by the method in association with each other. For example, in the above-described example, by describing "identification model: insertion method X, state transition model: insertion method Y" or the like, it is possible to indicate association between the method of computing the likelihood and the determined insertion method.

<Specific Method for Computing Likelihood>

As described above, for example, the determination unit 2040 computes the likelihood that the insertion method is used for each of the plurality of insertion methods in order to determine the insertion method being used. Hereinafter, as a method of computing this likelihood, two methods will be exemplified as specific examples.

<<Method Using Identification Model>>

In this method, an identification model that is trained to output the likelihood that each insertion method is being used in response to the shape data sequence 50 being input is utilized. Hereinafter, this identification model is referred to as an insertion method identification model. For example, the insertion method identification model is implemented by using a recurrent nueral network (RNN) or the like which is a neural network that handles time-series data. However, the type of the model used for implementing the insertion method identification model is not limited to the RNN, and any type of model that can handle the time-series data can be used.

The training of the insertion method identification model is performed using training data including a combination of "data representing time-series change in shape of endoscope scope, and ground-truth output data". The ground-truth output data is, for example, data representing the maximum likelihood (for example, 1) for the insertion method being used and representing the minimum likelihood (for example, 0) for the other insertion methods.

Note that data other than the shape data sequence 50 may be further input to the insertion method identification model. Examples of the data other than the shape data sequence 50 include still image data or video data obtained from the camera 10, data (for example, time-series data of acceleration obtained from an acceleration sensor attached to the hand of the operator, and the like) representing the motion of the operator of the endoscope scope 40, and the like. Furthermore, in a case where the shape data sequence 50 represents the shape of the endoscope scope 40 by a shape category, three-dimensional coordinates of each of a plurality of locations of the endoscope scope 40 may be further input to the insertion method identification model. When these data are used, the same type of data is also included in the training data of the insertion method identification model. In this way, the insertion method identification model is trained to compute the likelihood by further using data other than the shape data sequence 50.

Note that the insertion method identification model may output a label representing the insertion method being used, instead of outputting the likelihood for each insertion method. In this case, for example, the insertion method identification model outputs the label representing the insertion method for which the maximum likelihood is computed as a label of the insertion method being used. The determination unit 2040 can determine the insertion method being used by the label output from the insertion method specific model.

<<Method Using State Transition Model>>

In this case, for each of the plurality of insertion methods, the state transition model representing a temporal change pattern of the shape of the endoscope scope 40 in a case where the insertion method is used is determined and stored in advance in a storage device accessible from the determination unit 2040. When this method is used, the likelihood that each insertion method is used represents, for example, a degree of matching between the state transition model of the insertion method and the temporal change of the endoscope scope 40 represented by the shape data sequence 50. That is, for each of the plurality of insertion methods, the determination unit 2040 computes the degree to which the state transition model of the insertion method matches the shape data sequence 50, and determines the insertion method being used based on the computation result. For example, the insertion method with the maximum degree of the matching is determined as the insertion method being used.

In a case where the shape data 52 represents the shape category, for example, the likelihood is determined as follows using the degree of matching between the state transition model and the shape data sequence 50.
Equation 1

$$likelihood = \frac{number\ of\ shape\ categories\ matching\ each\ other}{unit\ period\ length} \quad (1)$$

In Equation (1), the numerator on the right side represents the number of shape categories that match between the shape data sequence 50 and the state transition model. Meanwhile, the unit period length of the denominator represents the total number of shape categories included in the unit period described above.

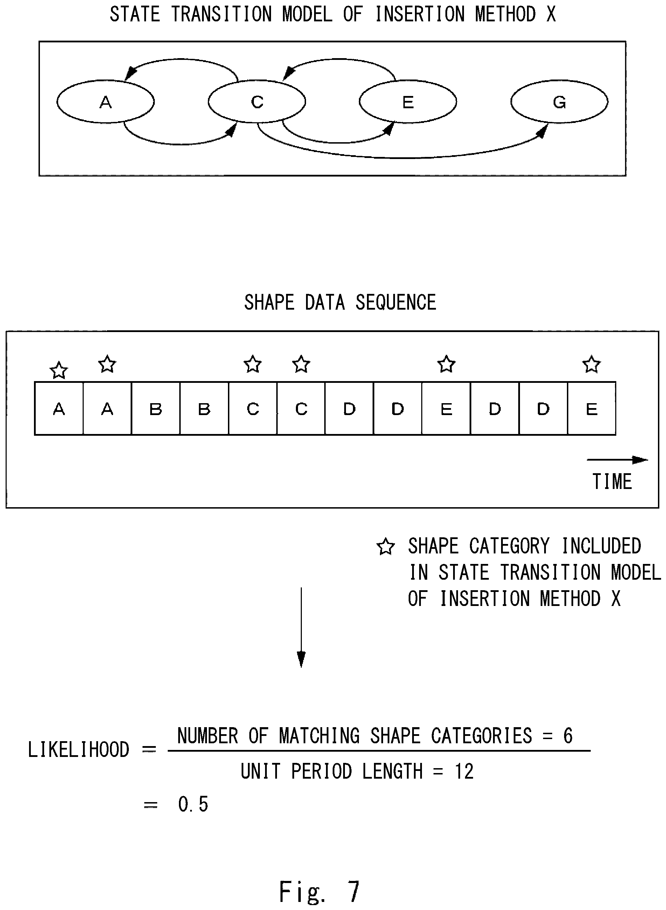

Fig. 7 is a diagram conceptually illustrating a method of computing the likelihood using Equation (1). In this example, the state transition model of the insertion method X is compared with the shape data sequence 50. In this example, the unit period length is 12.

The state transition model of the insertion method X includes four shape categories A, C, E, and G. The shape data sequence 50 includes two shape categories A, two shape categories C, and two shape categories E among the shape categories constituting the state transition model of the insertion method X. From this, the number of shape categories that match between the state transition model of the insertion method X and the shape data sequence 50 is 6. Therefore, according to Equation (1), 0.5 (6/12) is computed as the likelihood that the insertion method X is used.

A formula for computing the likelihood is not limited to Equation (1). For example, the likelihood may be computed using the following Equation (2).
Equation 2

*likelihood*

$$= \frac{number\ of\ shape\ categories\ matching\ each\ other}{total\ number\ of\ types\ of\ shape\ categories\ included\ in\ unit\ peiord} \quad (2)$$

For example, in the example of Fig. 7, five types of shape categories A, B, C, D, and E are included in the shape data sequence 50 of the unit period. Meanwhile, there are three types of shape categories A, C, and E that match each other between the state transition model of the insertion method X and the shape data sequence 50. Therefore, according to Equation (2), 0.6 (3/5) is computed as the likelihood that the insertion method X is used.

Furthermore, when computing the likelihood for each insertion method, the likelihood may be computed in consideration of the order of transitions by adding a computation formula that increases the likelihood in a case of following the transition path (order relation) indicated in the state transition model or decreases the likelihood in a case of not following the transition path, in addition to the degree of matching of the shape categories included in the unit period.

<Output of Insertion Method Information 20: S108>

The output unit 2060 outputs the insertion method information 20. There are various methods for outputting the insertion method information 20. For example, the output unit 2060 displays a screen representing the insertion method information 20 on a display device that can be viewed by the operator of the endoscope scope 40. In another example, the output unit 2060 may put a file representing the insertion method information 20 into a storage device or transmit the file to another arbitrary device.

Various pieces of information can be generated as the insertion method information 20. For example, the insertion method information 20 is information representing the name of the insertion method being used. In another example, the insertion method information 20 may indicate a transition (a temporal change of the insertion method being used) of the insertion method being used.

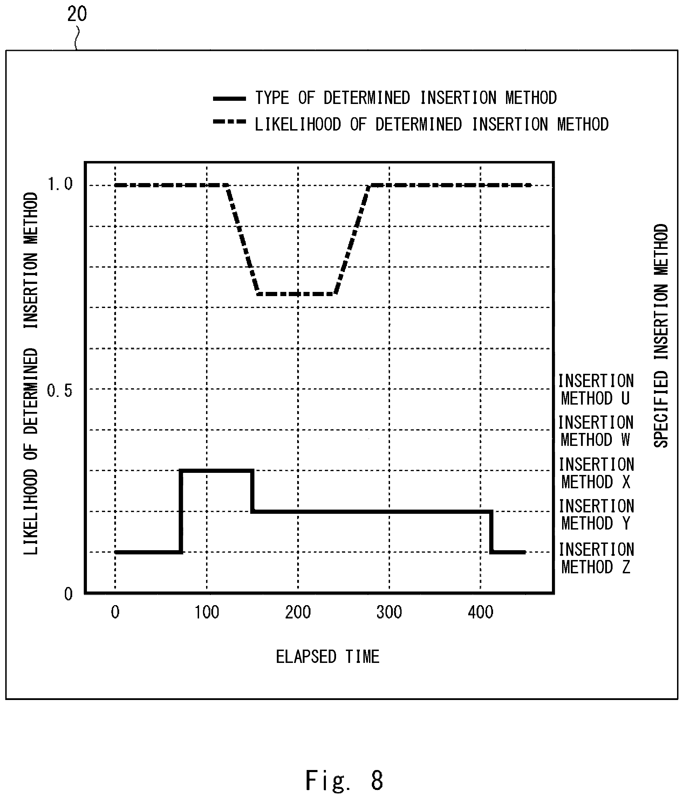

Fig. 8 is a diagram illustrating the insertion method information 20. In the insertion method information 20 of Fig. 8, two types of graphs are illustrated. The solid line represents the transitions of the insertion methods used for insertion of the endoscope scope 40 over time, which are determined by the endoscope operation support apparatus 2000. As described above, the insertion method used to insert the endoscope scope 40 may change over time. Therefore, in the example illustrated in Fig. 8, the endoscope operation support apparatus 2000 determines the insertion method used in the unit period for each unit period. Then, the determined insertion method is plotted in association with the time point (for example, the start time point of the unit period) corresponding to the unit period. In the example of Fig. 8, the insertion methods being used transition in the order of the insertion method Z, the insertion method X, the insertion method Y, and the insertion method Z.

An alternate long and short dash line represents the likelihood of the determined insertion method. For example, in a period in which the insertion method Z is determined as the insertion method being used, the likelihood that the insertion method Z is being used is represented. Similarly, in a period in which the insertion method X is determined as the insertion method being used, the likelihood that the insertion method X is being used is represented.

Although the present invention has been described above with reference to the example embodiments, the present invention is not limited to the above example embodiments. Various modifications that can be understood by those skilled in the art can be made to the configuration and details of the present invention within the scope of the present invention.

In the above example, the program can be stored using various types of non-transitory computer readable medium and provided to the computer. Non-transitory computer readable medium includes various types of tangible storage media. Examples of the non-transitory computer readable medium include a magnetic recording medium (for example, a flexible disk, a magnetic tape, or a hard disk drive), a magneto-optical recording medium (for example, a magneto-optical disk), a CD-ROM, a CD-R, a CD-R/W, and a semiconductor memory (for example, mask ROM, PROM (Programmable ROM), EPROM (Erasable PROM), flash ROM, and RAM). In addition, the program may be provided to the computer by various types of transitory computer readable medium. Examples of transitory computer readable medium include electrical signals, optical signals, and electromagnetic waves. The transitory computer readable medium can supply the program to the computer via a wired communication path such as an electric wire and an optical fiber, or a wireless communication path.

Some or all of the above example embodiments may be described as the following supplementary notes, but are not limited to the following.

(supplementary note 1)

An endoscope operation support apparatus comprising:

an acquisition unit configured to acquire a shape data sequence representing a temporal change of shape data of an endoscope scope;
a determination unit configured to determine an insertion method used for insertion of the endoscope scope based on the shape data sequence; and
an output unit configured to output insertion method information regarding the determined insertion method.

(supplementary note 2)

The endoscope operation support apparatus according to Supplementary note 1, wherein the determination unit computes, for each of a plurality of insertion methods of the endoscope scope, likelihood that the insertion method is used based on the shape data sequence, and determines the insertion method being used based on the computed likelihood.

(supplementary note 3)

The endoscope operation support apparatus according to Supplementary note 2, wherein the determination unit acquires, for each of the plurality of insertion methods, a state transition model representing a pattern of a temporal change of a shape of the endoscope scope in a case where the insertion method is used, and computes likelihood that the insertion method is used based on a degree of matching between the state transition model and the shape data sequence.

(supplementary note 4)

The endoscope operation support apparatus according to Supplementary note 2, wherein the determination unit determines the likelihood that each insertion method is used by inputting the shape data sequence to a trained identification model that is configured to output the likelihood that each of the plurality of insertion methods is used in response to an input of the data sequence representing the temporal change of the shape data of the endoscope scope.

(supplementary note 5)

The endoscope operation support apparatus according to any one of Supplementary notes 1 to 4, wherein the determination unit performs:

determining a plurality of candidates for an insertion method being used based on likelihood that each insertion method is used; and
determining the insertion method being used from among the plurality of candidates of the insertion method based on a priority defined for each insertion method.

(supplementary note 6)

The endoscope operation support apparatus according to any one of Supplementary notes 1 to 4, wherein the determination unit performs:

determining a plurality of candidates for the insertion method being used based on likelihood that each insertion method is used; and
determining the insertion method being used from among the plurality of candidates for the insertion method using information regarding an operator of the endoscope scope.

(supplementary note 7)

The endoscope operation support apparatus according to Supplementary note 6, wherein the information regarding the operator of the endoscope scope indicates one or more insertion methods used by the operator, or indicates a technical level of the operator regarding operation of the endoscope scope.

(supplementary note 8)

The endoscope operation support apparatus according to Supplementary note 1, wherein the determination unit determines the insertion method being used by inputting the shape data sequence to a trained identification model that is configured to output data representing the insertion method being used in response to the input of the data sequence representing the temporal change of the shape data of the endoscope scope.

(supplementary note 9)

The endoscope operation support apparatus according to any one of Supplementary notes 1 to 8, wherein the output unit outputs the insertion method information including transitions of insertion methods being used over time.

(supplementary note 10)

The endoscope operation support apparatus according to any one of Supplementary notes 1 to 9,

wherein a plurality of shape categories, which are types of shapes taken by the endoscope scope, are defined, and wherein each piece of the shape data included in the shape data sequence indicates one of the plurality of shape categories of the endoscope scope.

(supplementary note 11)

A control method executed by a computer, the control method comprising:

an acquisition step of acquiring a shape data sequence representing a temporal change of shape data of an endoscope scope;
a determining step of determining an insertion method used for insertion of the endoscope scope based on the shape data sequence; and
an output step of outputting insertion method information regarding the determined insertion method.

(supplementary note 12)

The control method according to Supplementary note 11, wherein, in the determining step, for each of a plurality of insertion methods of the endoscope scope, likelihood that the insertion method is used is computed based on the shape data sequence, and the insertion method being used is determined based on the computed likelihood.

(supplementary note 13)

The control method according to Supplementary note 12, wherein, in the determining step, for each of the plurality of insertion methods, a state transition model representing a pattern of a temporal change in a shape of the endoscope scope in a case where the insertion method is used is acquired, and the likelihood that the insertion method is used is computed based on a degree of matching between the state transition model and the shape data sequence.

(supplementary note 14)

The control method according to Supplementary note 12, wherein in the determining step, the likelihood that each insertion method is used is determined by inputting the shape data sequence to a trained identification model that is configured to output the likelihood that each of the plurality of insertion methods is used in response to an input of the data sequence representing the temporal change of the shape data of the endoscope scope.

(supplementary note 15)

The control method according to any one of Supplementary notes 11 to 14, wherein in the determining step,

a plurality of candidates for an insertion method being used are determined based on likelihood that each insertion method is used, and
an insertion method being used is determined from among the plurality of candidates for the insertion method based on a priority defined for each insertion method.

(supplementary note 16)

The control method according to any one of Supplementary notes 11 to 14, wherein in the determining step,

a plurality of candidates for an insertion method being used are determined based on likelihood that each insertion method is used, and
an insertion method being used is determined from among the plurality of candidates for the insertion method using information regarding the operator of the endoscope scope.

(supplementary note 17)

The control method according to Supplementary note 16, wherein the information regarding the operator of the endoscope scope indicates one or more insertion methods used by the operator, or indicates a technical level of the operator regarding an operation of the endoscope scope.

(supplementary note 18)

The control method according to Supplementary note 11, wherein in the determining step, an insertion method being used is determined by inputting a shape data sequence to a trained identification model that is configured to output data representing the insertion method being used in response to an input of the data sequence representing the temporal change of the shape data of the endoscope scope.

(supplementary note 19)

The control method according to any one of Supplementary notes 11 to 18, wherein in the outputting step, the insertion method information including transitions of the insertion methods being used over time is output.

(supplementary note 20)

The control method according to any one of Supplementary notes 11 to 19,

wherein a plurality of shape categories, which are types of shapes taken by the endoscope scope, are defined, and
wherein each piece of the shape data included in the shape data sequence indicates one of the plurality of shape categories of the endoscope scope.

(supplementary note 21)

A computer readable medium storing a program, the program causes a computer to execute:

an acquisition step of acquiring a shape data sequence representing a temporal change of shape data of an endoscope scope;
a determining step of determining an insertion method used for insertion of the endoscope scope based on using the shape data sequence; and
an output step of outputting insertion method information regarding the determined insertion method.

(supplementary note 22)

The computer readable medium according to Supplementary note 21, wherein, in the determining step, for each of a plurality of insertion methods of the endoscope scope, likelihood that the insertion method is used is computed based on the shape data sequence, and the insertion method being used is determined based on the computed likelihood.

(supplementary note 23)

The computer readable medium according to Supplementary note 22, wherein, in the determining step, for each of the plurality of insertion methods, a state transition model representing a pattern of a temporal change in a shape of the endoscope scope in a case where the insertion method is used is acquired, and the likelihood that the insertion method is used is computed based on a degree of matching between the state transition model and the shape data sequence.

(supplementary note 24)

The computer readable medium according to Supplementary note 22, wherein, in the determining step, the likelihood that each insertion method is used is determined by inputting the shape data sequence to a trained identification model that is configured to output the likelihood that each of the plurality of insertion methods is used in response to the input of the data sequence representing the temporal change of the shape data of the endoscope scope.

(supplementary note 25)

The computer readable medium according to any one of Supplementary notes 21 to 24, wherein in the determining step,

a plurality of candidates for an insertion method being used are determined based on likelihood that each insertion method is used, and
an insertion method being used is determined from among the plurality of candidates for the insertion method based on a priority defined for each insertion method.

(supplementary note 26)

The computer readable medium according to any one of Supplementary notes 21 to 24, wherein in the determining step,

a plurality of candidates for an insertion method being used are determined based on likelihood that each insertion method is used, and
an insertion method being used is determined from among the plurality of candidates for the insertion method using information regarding an operator of the endoscope scope.

(supplementary note 27)

The computer readable medium according to Supplementary note 26, wherein the information regarding the operator of the endoscope scope indicates one or more insertion methods used by the operator, or indicates a technical level of the operator regarding an operation of the endoscope scope.

(supplementary note 28)

The computer readable medium according to Supplementary note 21, wherein, in the determining step, an insertion method being used is determined by inputting a shape data sequence to a trained identification model that is configured to output data representing the insertion method being used in response to an input of a data sequence representing the temporal change of the shape data of the endoscope scope.

(supplementary note 29)

The computer readable medium according to any one of Supplementary notes 21 to 28, wherein in the outputting step, the insertion method information including transitions of the insertion methods being used over time is output.

(supplementary note 30)

The computer readable medium according to any one of Supplementary notes 21 to 29,

wherein a plurality of shape categories, which are types of shapes taken by the endoscope scope, are defined, and
wherein each piece of the shape data included in the shape data sequence indicates one of the plurality of shape categories of the endoscope scope.

(supplementary note 31)

A program causing a computer to execute:

an acquisition step of acquiring a shape data sequence representing a temporal change of shape data of an endoscope scope;
a determining step of determining an insertion method used for insertion of the endoscope scope based on the shape data sequence; and
an output step of outputting insertion method information regarding the determined insertion method.

(Supplementary Note 32)

The program according to Supplementary Note 31, wherein, in the determining step, for each of a plurality of insertion methods of the endoscope scope, likelihood that the insertion method is used is computed based on the shape data sequence, and the insertion method being used is determined based on the computed likelihood.

(Supplementary Note 33)

The program according to Supplementary Note 32, wherein, in the determining step, for each of the plurality of insertion methods, a state transition model representing a pattern of a temporal change in a shape of the endoscope scope in a case where the insertion method is used is acquired, and the likelihood that the insertion method is used is computed based on a degree of matching between the state transition model and the shape data sequence.

(Supplementary Note 34)

The program according to Supplementary Note 32, wherein, in the determining step, the likelihood that each insertion method is used is determined by inputting the shape data sequence to a trained identification model that is configured to output the likelihood that each of the plurality of insertion methods is used in response to the input of the data sequence representing the temporal change of the shape data of the endoscope scope.

(Supplementary Note 35)

The program according to any one of Supplementary Notes 31 to 34, wherein in the determining step,

a plurality of candidates for an insertion method being used are determined based on likelihood that each insertion method is used, and
an insertion method being used is determined from among the plurality of candidates for the insertion method based on a priority defined for each insertion method.

(Supplementary Note 36)

The program according to any one of Supplementary Notes 31 to 34, wherein in the determining step,

a plurality of candidates for an insertion method being used are determined based on likelihood that each insertion method is used, and
an insertion method being used is determined from among the plurality of candidates for the insertion method using information regarding an operator of the endoscope scope.

(Supplementary Note 37)

The program according to Supplementary Note 36, wherein the information regarding the operator of the endoscope scope indicates one or more insertion methods used by the operator, or indicates a technical level of the operator regarding an operation of the endoscope scope.

(Supplementary Note 38)

The program according to Supplementary Note 31, wherein, in the determining step, an insertion method being used is determined by inputting a shape data sequence to a trained identification model that is configured to output data representing the insertion method being used in response to an input of a data sequence representing the temporal change of the shape data of the endoscope scope.

(Supplementary Note 39)

The program according to any one of Supplementary Notes 31 to 38, wherein in the outputting step, the insertion method information including transitions of the insertion methods being used over time is output.

(Supplementary Note 40)

The program according to any one of Supplementary Notes 31 to 39,

wherein a plurality of shape categories, which are types of shapes taken by the endoscope scope, are defined, and
wherein each piece of the shape data included in the shape data sequence indicates one of the plurality of shape categories of the endoscope scope.

**Reference Signs List**

| | |
|---|---|
| 10 | CAMERA |
| 20 | INSERTION METHOD INFORMATION |
| 40 | ENDOSCOPE SCOPE |
| 50 | SHAPE DATA SEQUENCE |
| 51 | TIME POINT |
| 52 | SHAPE DATA |
| 60 | ENDOSCOPE CONTROL APPARATUS |
| 500 | COMPUTER |
| 500 | Each Computer |
| 502 | BUS |
| 504 | PROCESSOR |
| 506 | MEMORY |
| 508 | STORAGE DEVICE |
| 510 | INPUT/OUTPUT INTERFACE |
| 512 | NETWORK INTERFACE |
| 2000 | ENDOSCOPE OPERATION SUPPORT APPARATUS |
| 2020 | ACQUISITION UNIT |
| 2040 | DETERMINATION UNIT |
| 2060 | OUTPUT UNIT |

**Claims**

1. An endoscope operation support apparatus comprising:

   an acquisition unit configured to acquire a shape data sequence representing a temporal change of shape data of an endoscope scope;
   a determination unit configured to determine an insertion method used for insertion of the endoscope scope based on the shape data sequence; and
   an output unit configured to output insertion method information regarding the determined insertion method.

2. The endoscope operation support apparatus according to Claim 1, wherein the determination unit computes, for each of a plurality of insertion methods of the endoscope scope, likelihood that the insertion method is used based on the shape data sequence, and determines the insertion method being used based on the computed likelihood.

3. The endoscope operation support apparatus according to Claim 2, wherein the determination unit acquires, for each of the plurality of insertion methods, a state transition model representing a pattern of a temporal change of a shape of the endoscope scope in a case where the insertion method is used, and computes likelihood that the insertion method is used based on a degree of matching between the state transition model and the shape data sequence.

4. The endoscope operation support apparatus according to Claim 2, wherein the determination unit determines the likelihood that each insertion method is used by inputting the shape data sequence to a trained identification model that is configured to output the likelihood that each of the plurality of insertion methods is used in response to an input of the data sequence representing the temporal change of the shape data of the endoscope scope.

5. The endoscope operation support apparatus according to any one of Claims 1 to 4, wherein the determination unit performs:

determining a plurality of candidates for an insertion method being used based on likelihood that each insertion method is used; and
determining the insertion method being used from among the plurality of candidates of the insertion method based on a priority defined for each insertion method.

6. The endoscope operation support apparatus according to any one of Claims 1 to 4, wherein the determination unit performs:

determining a plurality of candidates for the insertion method being used based on likelihood that each insertion method is used; and
determining the insertion method being used from among the plurality of candidates for the insertion method using information regarding an operator of the endoscope scope.

7. The endoscope operation support apparatus according to Claim 6, wherein the information regarding the operator of the endoscope scope indicates one or more insertion methods used by the operator, or indicates a technical level of the operator regarding operation of the endoscope scope.

8. The endoscope operation support apparatus according to Claim 1, wherein the determination unit determines the insertion method being used by inputting the shape data sequence to a trained identification model that is configured to output data representing the insertion method being used in response to the input of the data sequence representing the temporal change of the shape data of the endoscope scope.

9. The endoscope operation support apparatus according to any one of Claims 1 to 8, wherein the output unit outputs the insertion method information including transitions of insertion methods being used over time.

10. The endoscope operation support apparatus according to any one of Claims 1 to 9,

wherein a plurality of shape categories, which are types of shapes taken by the endoscope scope, are defined, and
wherein each piece of the shape data included in the shape data sequence indicates one of the plurality of shape categories of the endoscope scope.

11. A control method executed by a computer, the control method comprising:

an acquisition step of acquiring a shape data sequence representing a temporal change of shape data of an endoscope scope;
a determining step of determining an insertion method used for insertion of the endoscope scope based on the shape data sequence; and
an output step of outputting insertion method information regarding the determined insertion method.

12. The control method according to Claim 11, wherein, in the determining step, for each of a plurality of insertion methods of the endoscope scope, likelihood that the insertion method is used is computed based on the shape data sequence, and the insertion method being used is determined based on the computed likelihood.

13. The control method according to Claim 12, wherein, in the determining step, for each of the plurality of insertion methods, a state transition model representing a pattern of a temporal change in a shape of the endoscope scope in a case where the insertion method is used is acquired, and the likelihood that the insertion method is used is computed based on a degree of matching between the state transition model and the shape data sequence.

14. The control method according to Claim 12, wherein in the determining step, the likelihood that each insertion method is used is determined by inputting the shape data sequence to a trained identification model that is configured to output the likelihood that each of the plurality of insertion methods is used in response to an input of the data sequence representing the temporal change of the shape data of the endoscope scope.

15. The control method according to any one of Claims 11 to 14, wherein in the determining step,

a plurality of candidates for an insertion method being used are determined based on likelihood that each insertion method is used, and
an insertion method being used is determined from among the plurality of candidates for the insertion method based on a priority defined for each insertion method.

16. The control method according to any one of Claims 11 to 14, wherein in the determining step,

a plurality of candidates for an insertion method being used are determined based on likelihood that each insertion method is used, and
an insertion method being used is determined from among the plurality of candidates for the insertion method using information regarding the operator of the endoscope scope.

17. The control method according to Claim 16, wherein the information regarding the operator of the endoscope scope indicates one or more insertion methods used by the operator, or indicates a technical level of the operator regarding an operation of the endoscope scope.

18. The control method according to Claim 11, wherein in the determining step, an insertion method being used is determined by inputting a shape data sequence to a trained identification model that is configured to output data representing the insertion method being used in response to an input of the data sequence representing the temporal change of the shape data of the endoscope scope.

19. The control method according to any one of Claims 11 to 18, wherein in the outputting step, the insertion method information including transitions of the insertion methods being used over time is output.

20. The control method according to any one of Claims 11 to 19,

wherein a plurality of shape categories, which are types of shapes taken by the endoscope scope, are defined, and
wherein each piece of the shape data included in the shape data sequence indicates one of the plurality of shape categories of the endoscope scope.

21. A computer readable medium storing a program, the program causes a computer to execute:

an acquisition step of acquiring a shape data sequence representing a temporal change of shape data of an endoscope scope;
a determining step of determining an insertion method used for insertion of the endoscope scope based on using the shape data sequence; and
an output step of outputting insertion method information regarding the determined insertion method.

22. The computer readable medium according to Claim 21, wherein, in the determining step, for each of a plurality of insertion methods of the endoscope scope, likelihood that the insertion method is used is computed based on the shape data sequence, and the insertion method being used is determined based on the computed likelihood.

23. The computer readable medium according to Claim 22, wherein, in the determining step, for each of the plurality of insertion methods, a state transition model representing a pattern of a temporal change in a shape of the endoscope scope in a case where the insertion method is used is acquired, and the likelihood that the insertion method is used is computed based on a degree of matching between the state transition model and the shape data sequence.

24. The computer readable medium according to Claim 22, wherein, in the determining step, the likelihood that each insertion method is used is determined by inputting the shape data sequence to a trained identification model that is configured to output the likelihood that each of the plurality of insertion methods is used in response to the input of the data sequence representing the temporal change of the shape data of the endoscope scope.

**25.** The computer readable medium according to any one of Claims 21 to 24, wherein in the determining step,

a plurality of candidates for an insertion method being used are determined based on likelihood that each insertion method is used, and

an insertion method being used is determined from among the plurality of candidates for the insertion method based on a priority defined for each insertion method.

**26.** The computer readable medium according to any one of Claims 21 to 24, wherein in the determining step,

a plurality of candidates for an insertion method being used are determined based on likelihood that each insertion method is used, and

an insertion method being used is determined from among the plurality of candidates for the insertion method using information regarding an operator of the endoscope scope.

**27.** The computer readable medium according to Claim 26, wherein the information regarding the operator of the endoscope scope indicates one or more insertion methods used by the operator, or indicates a technical level of the operator regarding an operation of the endoscope scope.

**28.** The computer readable medium according to Claim 21, wherein, in the determining step, an insertion method being used is determined by inputting a shape data sequence to a trained identification model that is configured to output data representing the insertion method being used in response to an input of a data sequence representing the temporal change of the shape data of the endoscope scope.

**29.** The computer readable medium according to any one of Claims 21 to 28, wherein in the outputting step, the insertion method information including transitions of the insertion methods being used over time is output.

**30.** The computer readable medium according to any one of Claims 21 to 29,

wherein a plurality of shape categories, which are types of shapes taken by the endoscope scope, are defined, and

wherein each piece of the shape data included in the shape data sequence indicates one of the plurality of shape categories of the endoscope scope.

**31.** A program causing a computer to execute:

an acquisition step of acquiring a shape data sequence representing a temporal change of shape data of an endoscope scope;

a determining step of determining an insertion method used for insertion of the endoscope scope based on the shape data sequence; and

an output step of outputting insertion method information regarding the determined insertion method.

52 SHAPE DATA SEQUENCE 50

40

2000

ENDOSCOPE OPERATION
SUPPORT APPARATUS

DETERMINE INSERTION METHOD USED FOR INSERTION OF ENDOSCOPE SCOPE
40 BASED ON TEMPORAL CHANGE OF SHAPE OF ENDOSCOPE SCOPE 40

OUTPUT INSERTION METHOD INFORMATION 20
REGARDING DETERMINED INSERTION METHOD

20

CURRENT INSERTION METHOD IS
[SHAFT RETENTION SHORTENING METHOD]

INSERTION METHOD INFORMATION

Fig. 1

2000

ENDOSCOPE OPERATION SUPPORT APPARATUS

2020

ACQUISITION UNIT

2040

DETERMINATION UNIT

2060

OUTPUT UNIT

Fig. 2

60

ENDOSCOPE
CONTROL APPARATUS

500

COMPUTER

506

MEMORY

510

INPUT/OUTPUT
I/F

502

BUS

PROCESSOR

STORAGE
DEVICE

NETWORK I/F

504

512

508

Fig. 3

```
                                40
                               ⌇
   ┌─────────────────────────────┐
   │           SCOPE             │
   │   ┌─────────────────────┐   │
   │   │      CAMERA         │──┼── 10
   │   └─────────────────────┘   │
   └─────────────┬───────────────┘
                 │
                                60
                               ⌇
   ┌─────────────┴───────────────┐
   │    ENDOSCOPE  CONTROL       │
   │        APPARATUS            │
   └─────────────┬───────────────┘
                 │
                               2000
                               ⌇
   ┌─────────────┴───────────────┐
   │   ENDOSCOPE  OPERATION      │
   │   SUPPORT  APPARATUS        │
   └─────────────────────────────┘
```

Fig. 4

```
                    ┌──────────────────┐
                    │      start       │
                    └──────────────────┘
                             │
   ┌─────────────────────────────────────────────────┐
   │      ACQUIRE SHAPE DATA SEQUENCE 50             │ ~ S102
   └─────────────────────────────────────────────────┘
                             │
   ┌─────────────────────────────────────────────────┐
   │      USE SHAPE DATA SEQUENCE 50 AND             │ ~ S104
   │      DETERMINE USED INSERTION METHOD           │
   └─────────────────────────────────────────────────┘
                             │
   ┌─────────────────────────────────────────────────┐
   │   OUTPUT INSERTION METHOD INFORMATION 20       │ ~ S106
   │   REGARDING DETERMINED INSERTION METHOD        │
   └─────────────────────────────────────────────────┘
                             │
                             ▼
                    ┌──────────────────┐
                    │       end        │
                    └──────────────────┘
```

Fig. 5

50

| TIME POINT | SHAPE DATA |
|:---:|:---:|
| t1 | CATEGORY E |
| t2 | CATEGORY B |
| . . . | . . . |

Fig. 6

STATE TRANSITION MODEL OF INSERTION METHOD X

A → C → E → G

SHAPE DATA SEQUENCE

| ☆ | ☆ |  |  | ☆ | ☆ |  |  | ☆ |  |  | ☆ |
| A | A | B | B | C | C | D | D | E | D | D | E |

→ TIME

☆ SHAPE CATEGORY INCLUDED
IN STATE TRANSITION MODEL
OF INSERTION METHOD X

$$\text{LIKELIHOOD} = \frac{\text{NUMBER OF MATCHING SHAPE CATEGORIES} = 6}{\text{UNIT PERIOD LENGTH} = 12}$$

$$= 0.5$$

Fig. 7

20

Fig. 8

## INTERNATIONAL SEARCH REPORT

| | International application No. |
|---|---|
| | PCT/JP2020/039529 |

**A. CLASSIFICATION OF SUBJECT MATTER**
Int. Cl. A61B1/00(2006.01)i
FI: A61B1/00 552

According to International Patent Classification (IPC) or to both national classification and IPC

**B. FIELDS SEARCHED**

Minimum documentation searched (classification system followed by classification symbols)
Int. Cl. A61B1/00-1/32

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2019/008726 A1 (OLYMPUS CORP.) 10 January 2019 (2019-01-10), claims, paragraphs [0060], [0063] | 1-3, 6-7, 9-13, 16-17, 19-23, 26-27, 29-31 |
| Y | | 4, 8, 14, 18, 24, 28 |
| A | | 5, 15, 25 |
| Y | WO 2016/207973 A1 (OLYMPUS CORP.) 29 December 2016 (2016-12-29), claims, paragraphs [0042]-[0048] | 4, 8, 14, 18, 24, 28 |
| A | WO 2016/135966 A1 (OLYMPUS CORP.) 01 September 2016 (2016-09-01), claims, all drawings | 1-31 |

☒ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01.12.2020 | 15.12.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/039529

C (Continuation). DOCUMENTS CONSIDERED TO BE RELEVANT

| Category* | Citation of document, with indication, where appropriate, of the relevant passages | Relevant to claim No. |
|---|---|---|
| A | JP 2015-196075 A (UNIVERSITY OF OCCUPATIONAL AND ENVIRONMENTAL HEALTH, JAPAN) 09 November 2015 (2015-11-09), claims, all drawings | 1-31 |
| A | WO 2019/003272 A1 (OLYMPUS CORP.) 03 January 2019 (2019-01-03), claims, all drawings | 1-31 |
| A | WO 2018/235185 A1 (OLYMPUS CORP.) 27 December 2018 (2018-12-27), claims, all drawings | 1-31 |
| A | WO 2018/069992 A1 (OLYMPUS CORP.) 19 April 2018 (2018-04-19), claims, all drawings | 1-31 |
| A | WO 2004/039249 A1 (OLYMPUS CORP.) 13 May 2004 (2004-05-13), claims, all drawings | 1-31 |

Form PCT/ISA/210 (continuation of second sheet) (January 2015)

| International application No. |
| --- |
| PCT/JP2020/039529 |

| Patent Documents referred to in the Report | Publication Date | Patent Family | Publication Date |
| --- | --- | --- | --- |
| WO 2019/008726 A1 | 10.01.2019 | US 2020/0129043 A1 claims, paragraphs [0074], [0077] CN 110831476 A | |
| WO 2016/207973 A1 | 29.12.2016 | (Family: none) | |
| WO 2016/135966 A1 | 01.09.2016 | US 2017/0347916 A1 claims, figures CN 107249423 A | |
| JP 2015-196075 A | 09.11.2015 | (Family: none) | |
| WO 2019/003272 A1 | 03.01.2019 | US 2020/0121163 A1 claims, figures CN 110769737 A | |
| WO 2018/235185 A1 | 27.12.2018 | | |
| WO 2018/069992 A1 | 19.04.2018 | US 2019/0231444 A1 claims, figures | |
| WO 2004/039249 A1 | 13.05.2004 | US 2005/0228221 A1 claims, figures CN 1694643 A EP 1504712 A1 JP 2004-147778 A JP 2004-358095 A | |

Form PCT/ISA/210 (patent family annex) (January 2015)

29

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 2018235185 A